# EUROPÄISCHE PATENTSCHRIFT

(11) **EP 2 750 803 B1**
(45) Veröffentlichungstag und Bekanntmachung des Hinweises auf die Patenterteilung: **26.06.2019**
(21) Anmeldenummer: 12751346.3
(22) Anmeldetag: 30.08.2012
(51) Int. Cl.: B05C 17/00, B43M 11/06, B65D 47/42, A61M 35/00

(54) **APPLIKATIONSSTIFT**
APPLICATOR STICK
CRAYON D'APPLICATION

(30) Priorität: 31.08.2011 EP 11179551
(43) Veröffentlichungstag der Anmeldung: 09.07.2014
(73) Patentinhaber: Sika Technology AG, 6340 Baar (CH)
(72) Erfinder: BINTIG, Janine, CH-8053 Zürich (CH); BONNA, Edmund, FL-9494 Schaan (LI)
(74) Vertreter: Sika Patent Attorneys
(86) Internationale Anmeldenummer: PCT/EP2012/066852
(87) Internationale Veröffentlichungsnummer: WO 2013/030270

(56) Entgegenhaltungen:
- US-A- 3 152 352
- US-A- 3 720 341
- US-A- 3 764 796
- US-A- 4 572 689
- US-A- 4 957 385
- US-A1- 2002 076 255
- US-A1- 2003 015 557
- US-A1- 2005 175 786
- US-B1- 6 478 191

## Beschreibung

### Technisches Gebiet

Die Erfindung betrifft einen Applikationsstift zum Austrag einer in einer harten Ampulle gespeicherten Flüssigkeit, etwa eines Primers oder Aktivatormittels zur Vorbehandlung von mit einem Klebstoff zu versehenden Oberflächen. Ein derartiger Applikationsstift hat einen zur Aufnahme der Ampulle ausgebildeten hohlen Kunststoff-Stiftkörper mit einem geschlossenen und einem offenen Ende, dessen Wandung hinreichend flexibel ist, um die im Kunststoff-Stiftkörper aufgenommene Ampulle zu brechen und hierdurch einen Austritt der Flüssigkeit in das Innere des Kunststoff-Stiftkörpers zu bewirken, und einen auf dem offenen Ende des Kunststoff-Stiftkörpers sitzenden Applikatorkopf, der einen Schaumstoff, Filz oder Faserkörper aufweist.

### Stand der Technik

Aus US 2002/076255 A1 ist ein Applikator zum Auftragen einer Lösung auf die Haut eines Patienten bekannt. Der Applikator hat einen Schaumstoff-Pad zur Auftragung der Flüssigkeit auf den Patienten und eine Vorrichtung zur Brechung einer Ampulle im Innern des Applikators.

Vorbehandlungsmittel zur Erzeugung von Klebeverbindungen werden üblicherweise in Verpackungen angeboten, die aus einer Aluminiumflasche, einem Polyethylen(PE)-Napf und einem Schraubverschluss der Flasche aus Polypropylen (PP) bestehen. Im Bereich der Einmal-Anwendungen werden auch Aluminiumtuben oder Primer-Stifte und Aktivator-Pads mit jeweils sehr geringem Inhalt angeboten.

Auch Applikationsstifte der oben beschriebenen Art sind seit längerem im Handel und im praktischen Einsatz. Bei bestimmten Produkten dieser Art ist ein spezielles Werkzeug zum Zerbrechen der im Applikationsstift aufgenommen Ampulle zur Freisetzung der darin enthaltenen Flüssigkeit vorgesehen; vgl. etwa die WO 2000/038564. Mit diesem Werkzeug wird lokal starker Druck auf die Kunststoffwand des Stiftkörpers ausgeübt. Die Wandung muss lediglich einen Grad von Verformbarkeit aufweisen, der eine Einleitung der mit dem Werkzeug ausgeübten Kraft in die zerbrechliche Wandung der Ampulle (in der Regel aus Glas) ermöglicht, kann also relativ steif sein. Hierdurch wird vermieden, dass beim Zerbrechen der Ampulle entstehende Scherben den Stiftkörper durchdringen und zu Verletzungen des Benutzers führen können.

Aus Sicht der Erfinder ist jedoch ein zu steifer Stiftkörper unter mehreren Aspekten problematisch in der Handhabung. Insbesondere erfordert die Benutzung eines solchen Applikationsstiftes nahezu zwingend den Einsatz des erwähnten Werkzugs zum Zerbrechen der Ampulle, während einfachere Handhabungen (so etwa das Zerbrechen der Ampulle durch Biegen des Stiftes über eine Tischkante oder von Hand) ausscheiden.

Der Erfindung liegt daher die Aufgabe zugrunde, einen verbesserten Applikationsstift der gattungsgemäßen Art bereitzustellen, der insbesondere leicht, flexibel und sicher zu benutzen ist.

### Offenbarung der Erfindung

Diese Aufgabe wird durch einen Applikationsstift mit den Merkmalen des Anspruchs 1 gelöst. Zweckmäßige Fortbildungen des Erfindungsgedankens sind Gegenstand der abhängigen Ansprüche.

Die gemäß dem ersten Aspekt der Erfindung mit dem zusätzlichen Mantel verstärkte, gleichwohl aber flexibel bleibende Wandung des Applikationsstiftes bietet hohe Sicherheit bei der Handhabung und zugleich die Möglichkeit, durch aus Übung gezielten Drucks den Produktfluss durch den Applikatorkopf zu verstärken bzw. in gewissem Grade zu steuern.

In einer Ausführung der Erfindung gemäß dem ersten Aspekt ist eine separate Mantelschicht vorgesehen, die zugleich als Beschriftungsträger dient. Da Applikationsstifte der genannten Art in der Regel zur Beschriftung mit einem Etikett versehen werden, ermöglicht dies die Realisierung der Erfindung praktisch ohne zusätzlichen Herstellungsaufwand.

In einer weiteren Ausführung erstreckt sich die separate Mantelschicht über den gesamten Griffbereich des Kunststoff-Stiftkörpers. Dies ermöglicht eine besonders flexible Handhabung des Stiftes gemäß der jeweiligen Fingerfertigkeit und Gewohnheiten des Benutzers unter Ausschluss von Verletzungsgefahren.

Eine weitere Ausführung sieht vor, dass die separate Mantelschicht klebend mit der Außenwandung des Kunststoff-Stiftkörpers verbunden ist. Hiermit entspricht die Aufbringung der Mantelschicht weitgehend derjenigen eines herkömmlichen Klebeetiketts und kann insbesondere mit verfügbaren Anlagen ausgeführt werden. Grundsätzlich ist jedoch auch die Verwendung eines Schrumpfschlauches oder eines gewöhnlichen aufziehbaren Schlauches mit lediglich lokaler Verklebung oder Verschweißung am primären Mantel des Stiftes möglich.

In einer weiteren Ausführung ist die separate Mantelschicht aus einer Polypropylen- oder ähnlichen Folie gebildet. Neben Polypropylen kommen etwa auch Materialien wie Polyethylen in Betracht.

In weiteren Ausführungen ist die separate Mantelschicht ein- oder mehrlagig (z. B. zweilagig) ausgebildet und hat eine Gesamtdicke im Bereich zwischen 80 und 160µm, insbesondere im Bereich zwischen 100 und 140µm. Je nach Wahl des speziellen Materials sowie des Materials und der Stärke der Wandung des eigentlichen Stiftkörpers können jedoch auch andere Wandstärken der separaten Mantelschicht zweckmäßig sein.

Der Kunststoff-Stiftkörper ist mindestens im Bereich seines offenen Endes zylindrisch geformt, und der Applikatorkopf weist eine Ringnut auf, in die das offene Ende des Kunststoff-Stiftkörpers eingreift. Hierbei sind an die Außenoberfläche und/oder die Innenoberfläche des Kunststoff-Stiftkörpers an oder nahe seinem offenen Ende Haltemittel mit Widerhaken-Effekt zum Festhalten des Applikatorkopfes auf dem Kunststoff-Stiftkörper und/oder der Ampulle im Kunststoff-Stiftkörper gegenüber einer axial nach vorn wirkenden Kraft angeformt.

Vorsprünge im Inneren des Stiftkörpers verhindern insbesondere, dass der Benutzer eine einmal eingeführte Ampulle wieder aus dem Stift herauszieht (und sich dabei verletzen könnte), zugleich aber auch ein unbeabsichtigtes Herausfliegen der Ampulle aus dem vorderen Ende des Stiftes, falls der Benutzer den Stift zum Befördern des Flüssigkeitsflusses ruckartig axial nach vorn bewegt. In ähnlicher Weise verhindern außen an den Stiftkörper angeformte Rasthaken oder ein Gewindeabschnitt, dass der Applikatorkopf sich bei ruckartigen Bewegungen des Stiftes vom Stiftkörper löst und hierbei verloren geht und die im Stiftkörper enthaltene Flüssigkeit unkontrolliert austreten lässt.

Der Gewindeabschnitt kann zum einen an das offene Ende des Stiftkörpers im Wesentlichen unter Beibehaltung von dessen normaler Wandstärke (und Flexibilität) angeformt sein. In einer anderen Ausführung können in den Gewindegängen Ausnehmungen vorgesehen sein, die die Elastizität des Vorderendes des Stiftkörpers wesentlich vergrößern. Hierdurch kann eine zusätzliche Saugwirkung bezüglich des im Inneren des Stiftkörpers vorhandenen Produkts und damit eine Förderung bzw. gewisse Steuerbarkeit des Produktflusses erreicht werden.

In einer weiteren Ausführung der Erfindung ist vorgesehen, dass der an die Außenoberfläche angeformte Gewindeabschnitt mit lokalen Abdünnungen über seinen gesamten Umfang geschlossen ist, derart, dass durch ihn keine Flüssigkeit austreten kann, bei weitgehender Bewahrung der Vorteile, die sich durch die vergrößerte Elastizität des offenen Stiftendes ergeben.

In einer weiteren Ausführungsform ist der Gewindeabschnitt zum Festhalten des Applikatorkopfes scharfkantig ausgeführt. Dies erleichtert das Einschneiden in das Material des Applikatorkopfes, wenn dieser auf den Stift aufgeschraubt wird, und erhöht die Haltekraft. Zusätzlich kann vorgesehen sein, dass der Neigungswinkel der Gewindeflanken zum offenen Ende des Stiftkörpers hin kleiner als zum geschlossenen Ende ist, womit dem Gewindeabschnitt ebenfalls eine Art Widerhaken-Charakteristik verliehen wird.

Zu dem erwähnten Gewindeabschnitt am Stiftkörper korrespondiert eine in den Applikatorkopf eingefräste Ringnut oder ein (leicht und kostengünstiger zu erzeugender) ringförmiger Schnitt, in die/den der Gewindeabschnitt beim Schrauben des Applikatorkopfes auf den Stiftkörper eingreift.

Die Haltemittel bzw. Vorsprünge zum Festhalten bzw. Sichern der Ampulle können durch von außen vorgenommene Einprägungen in die Wandung des Stiftkörpers gebildet sein, die einen kleinen, z. B. im wesentlichen streifenförmigen, Abschnitt des Wandungsmaterials (ohne zusätzlichen Materialeinsatz) in das erwähnte Haltemittel bzw. den Vorsprung umwandeln.

### Beschreibung der Zeichnungen

Vorteile und Zweckmäßigkeiten der Erfindung werden aus der nachfolgenden Beschreibung bevorzugter Ausführungsbeispiele anhand der Figuren deutlich. Von diesen zeigen:
- Fig. 1: in skizzenartiger perspektivischer Darstellung (Explosionsdarstellung) einen erfindungsgemäßen Applikationsstift,
- Fig. 2: eine weitere perspektivische Darstellung eines erfindungsgemäßen Applikationsstiftes, in Art einer "Röntgendarstellung",
- Fig. 3: eine Detailansicht des offenen Endes des Kunststoff-Stiftkörpers eines erfindungsgemäßen Applikationsstiftes,
- Fig. 4: eine Detailansicht des offenen Endes des Kunststoff-Stiftkörpers eines weiteren erfindungsgemäßen Applikationsstiftes und
- Fig. 5 und 5A: Querschnittsdarstellungen (Fig. 5A als Ausschnitt) des offenen Endes einer weiteren Ausführung des erfindungsgemäßen Applikationsstiftes.

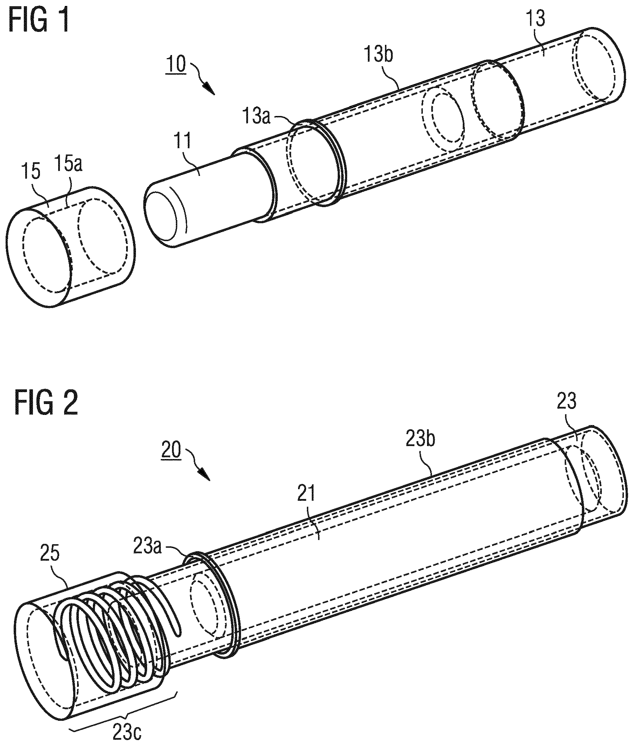

Fig. 1 zeigt einen Applikationsstift 10 zum flächigen Auftrag eines in einer Ampulle 11 verpackten Primers zur Vorbehandlung von Klebeflächen, welcher einen Kunststoff-Stiftkörper 13 und einen Schaumstoff-Applikatorkopf 15 umfasst. Die Ampulle 11 besteht aus Glas und der Kunststoff-Stiftkörper 13 etwa aus Polyamid (PA), PE oder PP. Eine ringförmige Rippe 13a am Stiftkörper 13 ist als Anschlag für den vom offenen Ende des Stiftkörpers her aufzuschiebenden Applikatorkopf 15 vorgesehen. Der vollständig aus Schaumstoff bestehende Applikatorkopf 15 hat ebenso wie der Stiftkörper 13 eine zylindrische Grundform. In ihm ist eine Ringnut 15a eingearbeitet, deren Durchmesser derjenigen des Stiftkörpers 13 entspricht, so dass das offene Ende des Stiftkörpers beim Aufsetzen des Applikatorkopfes in diese Ringnut eingreift. Im Übrigen sei angemerkt, dass mit dem Begriff "Applikationsstift" auch Applikatoren gemeint sind, die keine zylindrische Grundform haben, sondern etwa eine abgerundete prismatische, bauchige oder sonstiger andere, für den bestimmungsgemäßen Gebrauch aber geeignete Form aufweisen.

Im mittleren Bereich ist der Stiftkörper 13 mit einer PP-Klebefolie 13b zur Verstärkung seiner Durchstechfestigkeit umwickelt. Die PP-Klebefolie 13b wirkt als separate Mantelschicht nicht nur verstärkend, sondern dient zugleich als Beschriftungsträger von Produktkennzeichnungen, Herstellerangaben, Benutzungshinweisen etc. Sie ist in der dargestellten Ausführung einlagig und hat eine Stärke von 120µm, kann aber auch zweilagig aus einer 60µm-Folie gebildet sein.

Fig. 2 zeigt als modifizierte Ausführungsform, und zwar in Art einer Röntgendarstellung im zusammengesetzten Zustand, einen Applikationsstift 20. Mit der Ausführung nach Fig. 1 übereinstimmende oder gleichwirkende Teile sind mit an Fig. 1 angelehnten Bezugsziffern bezeichnet und werden hier nicht weiter erläutert. Die Konfiguration nach Fig. 2 unterscheidet sich von derjenigen nach Fig. 1 zunächst dadurch, dass die separate Mantelschicht 23b aus einer verstärkenden PP-Folie nahezu die gesamte Länge des aus einem "weichen" Kunststoff gefertigten Stiftkörpers 23 ummantelt. Zudem ist am offenen Ende des Stiftkörpers 23 ein Gewindeabschnitt 23c vorgesehen, der zum besseren Festhalten des Applikatorkopfes 25 auf dem Stiftkörper dient. Die ringförmige Rippe 23a ist so platziert, dass sie ein zu weites Verrutschen des Applikatorkopfes nach hinten bei starkem Aufdrücken seitens des Benutzers und entsprechender starker Verformung des elastischen Gewindeabschnitts 23c verhindert.

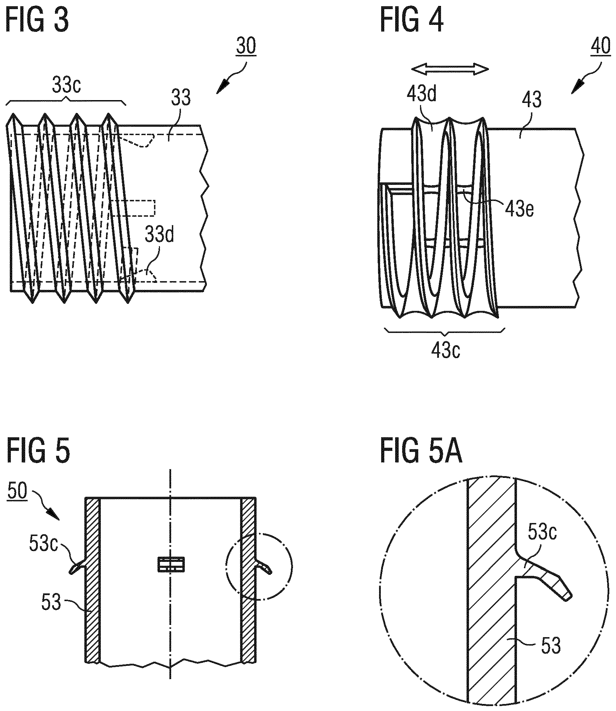

Fig. 3 zeigt in einer Detaildarstellung, die wiederum in Art einer Röntgenansicht gestaltet ist, das offene Ende des Kunststoff-Stiftkörpers 33 eines weiteren Applikationsstiftes 30. An der Außenoberfläche trägt der Stiftkörper wiederum einen Gewindeabschnitt 33c, der scharfkantig mit symmetrischer Geometrie der Gewindeflanken ausgeführt ist. An der Innenwandung des Stiftkörpers sind nach Innen weisende Vorsprünge 33d angeformt, die insofern "asymmetrisch" ausgeführt sind, als ihre zum offenen Ende des Stiftkörpers weisende Flanke bezüglich der Mantel-Innenoberfläche einen kleineren Anstiegswinkel als die dem geschlossenen Ende des Stiftkörpers zugewandte Flanke aufweist. Zudem sind die Vorsprünge 33d an ihrem Gipfelpunkt abgerundet. Die Vorsprünge 33d können daher, wenn eine (nicht dargestellte) Ampulle in den Stiftkörper eingeführt wird, von der Ampulle relativ leicht überwunden werden, wogegen sie die Ampulle bei Einwirkung von axial nach vorn wirkenden Kräfte sicher festhalten. Zudem ermöglicht die spezielle Form der Vorsprünge eine leichte Entformung aus dem Spritzgießwerkzeug, mit dem der Kunststoff-Stiftkörper hergestellt wird. Im Übrigen kann u. U. die Flexibilität des Stiftkörpers diese Entformung zusätzlich erleichtern.

Fig. 4 zeigt als weiter modifizierte Ausführung das vordere Ende des Stiftkörpers 43 eines weiteren Applikationsstiftes 40. Hier ist ein Gewindeabschnitt 43c vorgesehen, der durch ringförmige Abdünnungen 43d, zwischen denen einzelne stärkere Rippen 43e stehen bleiben, elastisch verformbar ist. Die durch diese Gewindekonfiguration geschaffene zusätzliche Elastizität des Stift-Vorderendes ermöglicht eine verbesserte Handhabung des Applikationsstiftes, speziell eine stärkere Komprimierung des Schaummaterials des Applikatorkopfes und hierdurch eine erhöhte Saugwirkung des Schaums für die nach Zerbrechen der Ampulle im Stiftinneren gesammelte Flüssigkeit, die mittels des Applikatorkopfes ausgetragen und auf eine Fläche verteilt wird. Andererseits verhindert die auch im Bereich der Abdünnungen vollständig geschlossene Umfangsfläche des Gewindeabschnitts einen unerwünschten seitlichen Austritt von im Stiftkörper befindlicher Flüssigkeit.

Fig. 5 und 5A zeigen das offene Ende des Stiftkörpers 53 eines weiteren Applikationsstiftes 50, bei dem an der Außenfläche einzelne nach hinten geneigte Rasthaken 53c die Funktion des bei den vorstehend beschriebenen Ausführungen vorgesehenen Gewindeabschnitts, also die Funktion des Festhaltens des aufgesteckten (nicht dargestellten) Applikatorkopfes, übernehmen. Fig. 5A zeigt die Geometrie dieser Rasthaken 53c in einer speziellen Ausgestaltung genauer. Die spezielle Geometrie ermöglicht ein gezieltes Nachgeben unter axial vom offenen Ende her wirkendem Druck (wie er beim Aufstecken des Applikatorkopfes auftritt), setzt aber andererseits einer in Richtung zum offenen Ende hin wirkenden Kraft einen relativ hohen Widerstand entgegen. Die Rasthaken erhöhen somit die Verliersicherheit des Applikatorkopfes auch bei "rauer" Handhabung durch den Benutzer.

Die Ausführung der Erfindung ist nicht auf diese Beispiele beschränkt, sondern ebenso in einer Vielzahl von Abwandlungen möglich, die im Rahmen fachgemäßen Handelns liegen.

## Patentansprüche

1. Applikationsstift (20 - 50) zum Austrag einer in einer harten Ampulle (21) gespeicherten Flüssigkeit, mit einem zur Aufnahme der Ampulle ausgebildeten hohlen Kunststoff-Stiftkörper (23; 33; 43; 53) mit einem geschlossenen und offenen Ende, dessen Wandung hinreichend flexibel ist, um die im Kunststoff-Stiftkörper aufgenommene Ampulle zu brechen und hierdurch einen Austritt der Flüssigkeit in das Innere des Kunststoff-Stiftkörpers zu bewirken, und einem auf dem offenen Ende des Kunststoff-Stiftkörpers sitzenden Applikatorkopf (15; 25), der einen Schaumstoff, Filz oder Faserkörper aufweist, wobei der Kunststoff-Stiftkörper mindestens im Bereich seines offenen Endes zylindrisch geformt ist und der Applikatorkopf (15; 25) eine Ringnut oder einen ringförmigen Schnitt (15a) aufweist, in die/den das offene Ende des Kunststoff-Stiftkörpers eingreift, **dadurch gekennzeichnet, dass** an die Außenoberfläche des Kunststoff-Stiftkörpers an oder nahe seinem offenen Ende Rasthaken (53c) zum Festhalten des Applikatorkopfes auf dem Kunststoff-Stiftkörper und/oder an die Innenoberfläche Vorsprünge zum Festhalten der Ampulle im Kunststoff-Stiftkörper gegenüber einer axial nach vorn wirkenden Kraft angeformt sind.

2. Applikationsstift nach Anspruch 1, wobei ein Gewindeabschnitt (33c; 34c) zum Festhalten des Applikationskopfes auf dem Kunststoff-Stiftkörper angeformt ist.

3. Applikationsstift nach Anspruch 2, wobei der an die Außenoberfläche angeformte Gewindeabschnitt (43c) durch lokale Abdünnungen (43d) oder Ausnehmungen in der Wandung elastisch verformbar ausgeführt ist.

4. Applikationsstift nach Anspruch 2, wobei der an die Außenoberfläche angeformte Gewindeabschnitt mit lokalen Abdünnungen über seinen gesamten Umfang geschlossen ist, derart, dass durch ihn keine Flüssigkeit austreten kann.

5. Applikationsstift nach einem der vorhergehenden Ansprüche, wobei die Rasthaken (53c) eine Widerhaken-Charakteristik aufweisen, derart, dass sie dem Aufsetzen des Applikatorkopfes relativ geringen Widerstand, einem Abziehen des Applikatorkopfes aber einen hohen Widerstand entgegensetzen.

6. Applikationsstift nach einem der Ansprüche 2 bis 5, wobei der Gewindeabschnitt (33c; 43c) zum Festhalten des Applikatorkopfes (15; 25) scharfkantig ausgeführt ist.

7. Applikationsstift nach einem der vorhergehenden Ansprüche, wobei die an die Innenwandung angeformten Vorsprünge eine abgerundete Sägezahnform aufweisen, wobei ein zum offenen Ende des Kunststoff-Stiftkörpers hin weisender Abschnitt einen geringeren Neigungswinkel als ein zum geschlossenen Ende hin weisender Abschnitt hat.

8. Applikationsstift nach einem der vorhergehenden Ansprüche, wobei die an die Innenwandung angeformten Vorsprünge durch Einprägungen von außen in die Wandung des Stiftkörpers gebildet sind.

9. Applikationsstift (10 - 50) nach einem der vorhergehenden Ansprüche, wobei der Kunststoff-Stiftkörper mindestens abschnittsweise eine separate Mantelschicht (13b) zur Erhöhung seiner Durchstechfestigkeit für Ampullen-Bruchstücke und nahe seinem offenen Ende Haltemittel zum Festhalten des Applikatorkopfes und/oder Haltemittel zum Festhalten der Ampulle in seinem Inneren aufweist.

10. Applikationsstift nach Anspruch 9, wobei die separate Mantelschicht (13b) zugleich als Beschriftungsträger dient.

11. Applikationsstift nach einem der Ansprüche 9 oder 10, wobei die separate Mantelschicht (13b) klebend mit der Außenwandung des Kunststoff-Stiftkörpers verbunden ist.

12. Applikationsstift nach einem der Ansprüche 9 bis 11, wobei die separate Mantelschicht (13b) aus einer Polypropylen oder ähnlichen Folie gebildet ist.

13. Applikationsstift nach einem der Ansprüche 9 bis 12, wobei die separate Mantelschicht (13b) ein- oder mehrlagig, insbesondere zweilagig, ausgebildet ist und eine Gesamtdicke im Bereich zwischen 80 und 160 µm, insbesondere im Bereich zwischen 100 und 140 µm, hat.

14. Applikationsstift nach einem der Ansprüche 9 bis 13, wobei die Haltemittel zum Festhalten der Ampulle als Vorsprünge an der Innenoberfläche ausgebildet sind, die insbesondere durch eine Einprägung von außen in die Mantelschicht gebildet sind.

## Claims

1. A stick applicator (20 - 50) for discharging a liquid stored in a hard ampule (21), having a hollow plastic stick body (23; 33; 43; 53) designed for receiving the ampule and including a closed and an open end, the wall of which is sufficiently flexible to rupture the ampule held in the plastic stick body and to, thereby cause the liquid to escape into the interior of the plastic stick body, and an applicator head (15; 25) which is attached to the open end of the plastic stick body and which comprises a foam, felt or fiber body, wherein the plastic stick body is cylindrically shaped at least in the region of its open end and the applicator head (15; 25) includes an annular groove or an annular cut (15a) into which the open end of the plastic stick body engages, **characterized in that** formed on the outer surface of the plastic stick body at or near the open end thereof are snap-in hooks (53c) for securely holding the applicator head on the plastic stick body and/or formed on the interior surface are projections for securely holding the ampule in the plastic stick body against an axially forward acting force.

2. The stick applicator according to claim 1, wherein a thread section (33c; 34c) for securely holding the applicator head on the plastic stick body is formed.

3. The stick applicator according to claim 2, wherein the thread section (43c) formed on the outer surface is designed to be elastically deformable as a result of locally thinned sections (43d) or recesses in the wall.

4. The stick applicator according to claim 2, wherein the thread section with locally thinned sections formed on the outer surface is closed over its entire circumference such that no liquid is able to escape through it.

5. The stick applicator according to one of the preceding claims, wherein the snap-in hooks (53c) exhibit a barbed-hook characteristic such that they offer relatively little resistance to the attachment of the applicator head, but offer strong resistance to a removal of the applicator head.

6. The stick applicator according to one of claims 2 to 5, wherein the thread section (33c; 43c) is designed with sharp edges for securely holding the applicator head (15; 25).

7. The stick applicator according to one of the preceding claims, wherein the projections formed on the interior wall have a rounded saw-toothed shape, a section pointing toward the open end of the plastic stick body having an angle of inclination smaller than a section pointing toward the closed end.

8. The stick applicator according to one of the preceding claims, wherein the projections formed on the interior wall are formed by impressions from the outside in the wall of the stick body.

9. The stick applicator (10 - 50) according to one of the preceding claims, wherein the plastic stick body includes at least in sections a separate sheathing layer (13b) for strengthening its perforation resistance to ampule fragments and near its open end retaining means for securely holding the applicator head and/or retaining means for securely holding the ampule in the interior thereof.

10. The stick applicator according to claim 9, wherein the separate sheathing layer (13b) functions simultaneously as an inscription carrier.

11. The stick applicator according to either of claims 9 and 10, wherein the separate sheathing layer (13b) is adhesively bonded to the outer wall of the plastic stick body.

12. The stick applicator according to one of claims 9 to 11, wherein the separate sheathing layer (13b) is formed from a polypropylene or similar film.

13. The stick applicator according to one of claims 9 to 12, wherein the separate sheathing layer (13b) is single or multi-layered, in particular double-layered, in design and has a total thickness in the range of between 80 µm and 160 µm, in particular in the range of between 100 µm and 140 µm.

14. The stick applicator according to one of claims 9 to 13, wherein the retaining means for securely holding the ampule are designed as projections on the interior surface, which are formed in particular from the outside by an impression in the sheathing layer.

## Revendications

1. Crayon d'application (20 - 50) pour décharger un liquide stocké dans une ampoule dure (21), comprenant un corps de crayon en plastique creux (23 ; 33 ; 43 ; 53) réalisé pour recevoir l'ampoule, avec une extrémité fermée et une extrémité ouverte, dont la paroi est suffisamment flexible pour rompre l'ampoule reçue dans le corps de crayon en plastique et pour provoquer de ce fait une expulsion du liquide à l'intérieur du corps de crayon en plastique, et une tête d'applicateur (15 ; 25) reposant sur l'extrémité ouverte du corps de crayon en plastique, qui présente une mousse, un feutre ou un corps fibreux, le corps de crayon en plastique étant formé au moins dans la région de son extrémité ouverte sous forme cylindrique et la tête d'applicateur (15 ; 25) présentant une rainure annulaire ou une découpe de forme annulaire (15a) dans laquelle s'engage l'extrémité ouverte du corps de crayon en plastique, **caractérisé en ce que** des crochets d'encliquetage (53c) pour fixer la tête d'applicateur sur le corps de crayon en plastique sont formés sur la surface extérieure du corps de crayon en plastique au niveau ou à proximité de son extrémité ouverte et/ou des saillies pour fixer l'ampoule dans le corps de crayon en plastique par rapport à une force agissant axialement vers l'avant sont formées sur la surface intérieure.

2. Crayon d'application selon la revendication 1, dans lequel une portion filetée (33c ; 34c) est formée pour fixer la tête d'applicateur sur le corps de crayon en plastique.

3. Crayon d'application selon la revendication 2, dans lequel la portion filetée (43c) formée sur la surface extérieure est réalisée de manière déformable élastiquement par des amincissements locaux (43d) ou des évidements dans la paroi.

4. Crayon d'application selon la revendication 2, dans lequel la portion filetée formée au niveau de la surface extérieure avec des amincissements locaux est fermée sur toute sa périphérie de telle sorte que du liquide ne puisse pas sortir à travers elle.

5. Crayon d'application selon l'une quelconque des revendications précédentes, dans lequel les crochets d'encliquetage (53c) présentent une caractéristique de barbillon telle qu'ils opposent une relativement faible résistance à la pose de la tête d'applicateur mais qu'ils opposent une grande résistance au retrait de la tête d'applicateur.

6. Crayon d'application selon l'une quelconque des revendications 2 à 5, dans lequel la portion filetée (33c ; 43c) est réalisée avec des arêtes vives pour fixer la tête d'applicateur (15 ; 25).

7. Crayon d'application selon l'une quelconque des revendications précédentes, dans lequel les saillies formées au niveau de la paroi interne présentent une forme en dents de scie arrondie, une portion tournée vers l'extrémité ouverte du corps de crayon en plastique présentant un angle d'inclinaison plus petit qu'une portion tournée vers l'extrémité fermée.

8. Crayon d'application selon l'une quelconque des revendications précédentes, dans lequel les saillies formées au niveau de la paroi interne sont formées par des gaufrages depuis l'extérieur dans la paroi du corps de crayon.

9. Crayon d'application (10-50) selon l'une quelconque des revendications précédentes, dans lequel le corps de crayon en plastique présente au moins en partie une couche d'enveloppe séparée (13b) pour augmenter sa résistance au perçage pour des pièces d'ampoule cassables et, à proximité de son extrémité ouverte, des moyens de retenue pour fixer la tête d'applicateur et/ou des moyens de retenue pour fixer l'ampoule à l'intérieur de celui-ci.

10. Crayon d'application selon la revendication 9, dans lequel la couche d'enveloppe séparée (13b) sert également de support d'écriture.

11. Crayon d'application selon l'une quelconque des revendications 9 ou 10, dans lequel la couche d'enveloppe séparée (13b) est connectée par collage à la paroi externe du corps de crayon en plastique.

12. Crayon d'application selon l'une quelconque des revendications 9 à 11, dans lequel la couche d'enveloppe séparée (13b) est formée de polypropylène ou d'une feuille similaire.

13. Crayon d'application selon l'une quelconque des revendications 9 à 12, dans lequel la couche d'enveloppe séparée (13b) est réalisée en une ou plusieurs couches, en particulier en deux couches et présente une épaisseur totale dans la plage comprise entre 80 et 160 µm, en particulier dans la plage comprise entre 100 et 140 µm.

14. Crayon d'application selon l'une quelconque des revendications 9 à 13, dans lequel les moyens de retenue pour fixer l'ampoule sont réalisés sous forme de saillies au niveau de la surface intérieure, lesquelles sont notamment formées par un gaufrage depuis l'extérieur dans la couche d'enveloppe.
